(19) Europäisches Patentamt
European Patent Office
Office européen des brevets

(11) **EP 4 566 514 A1**

(12) **EUROPEAN PATENT APPLICATION**

(43) Date of publication:
**11.06.2025 Bulletin 2025/24**

(21) Application number: **23215038.3**

(22) Date of filing: **07.12.2023**

(51) International Patent Classification (IPC):
**A61B 5/00** (2006.01)    **A61C 17/22** (2006.01)
**G06T 7/00** (2017.01)    **A46B 15/00** (2006.01)

(52) Cooperative Patent Classification (CPC):
**A61B 5/0088; A46B 13/02; A46B 15/0034;**
**A46B 15/0036; A61B 5/0071; A61B 5/4547;**
**A61C 17/221; G06T 7/0012;** A46B 15/004;
A46B 15/0044; A46B 2200/1066; G06T 2207/10024;
G06T 2207/30036

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Koninklijke Philips N.V.
5656 AG Eindhoven (NL)**

(72) Inventors:
• **KOOIJMAN, Gerben**
  **Eindhoven (NL)**
• **CIUHU, Calina**
  **Eindhoven (NL)**
• **GOTTENBOS, Bart**
  **Eindhoven (NL)**

(74) Representative: **Philips Intellectual Property &
Standards
High Tech Campus 52
5656 AG Eindhoven (NL)**

(54) **SYSTEM FOR DETECTING DENTAL PLAQUE**

(57)    A system and method are provided for detecting dental plaque. Intra-oral images are analyzed to detect image characteristics indicative of at least plaque and tartar. Tooth locations are detected at which the image characteristics are present; and a likelihood of plaque being present is derived based on the detected image characteristics and their associated tooth locations.

FIG. 2

EP 4 566 514 A1

**Description**

FIELD OF THE INVENTION

This invention relates to systems for detecting dental plaque, in particular based on image analysis. It for example relates to a detection system that may be integrated with an electric toothbrush.

BACKGROUND OF THE INVENTION

Oral hygiene is an important element in maintaining oral health. In addition to routine dentist visits, consumers express the need to monitor more frequently their oral health and receive guidance and reassurance on how well the teeth cleaning is performed.

It is known to use camera-based sensing to monitor oral hygiene and detect the development of actionable plaque as well as gum and tooth disease. For example, it has been proposed to use quantitative light-induced fluorescence (QLF) imaging.

QLF imaging can be used to detect plaque. However, a high fluorescence signal might equally indicate plaque or tartar. In providing feedback and guidance to the user, discriminating between the two is highly desired, as plaque is actionable (i.e., can be removed with brushing), whereas tartar is not.

SUMMARY OF THE INVENTION

The invention is defined by the claims.

According to examples in accordance with an aspect of the invention, there is provided a system for detecting dental plaque, comprising:

a camera system for generating intra-oral images;
a processor for processing the generated images, configured to:

detect image characteristics indicative of at least plaque and tartar;
from the generated images, detect tooth locations at which the image characteristics are present; and
estimate a likelihood of plaque being present based on the detected image characteristics and their associated tooth locations.

It is known to use image-based sensing (for example with a simple QLF camera) for the detection of plaque, tartar or other features, but without any discrimination technologies between the different features.

The invention is based on the recognition that an important factor to discriminate between (actionable) plaque and (unactionable) tartar is that their likelihood is strongly correlated with location. Here location can include dentition segment (e.g., 'inner right upper molars'), specific tooth (e.g., 'upper right 2nd molar'), and /or tooth region (e.g., 'close to gumline'). Therefore, tooth location can be used as a prior for conditional probability of either plaque or tartar being detected, thus improving the accuracy of plaque detection. Also, identifying tooth location enables better advice to be provided to a consumer (e.g., 'brush front teeth better at the gumline').

The system of the invention thus enables users to have an improved experience as the system can provide a plaque indication when plaque is detected, but not when there is a very high likelihood that the detected image characteristics relate to tartar. This avoids the system advising the user that there are plaque areas, but the user will not be able to brush them away because they are tartar.

The image characteristics may, in addition to tartar and plaque, be indicative of a stained spot, carious lesion or crack. Thus, while the system of the invention is of primary interest for discriminating between plaque and tartar it also can estimate a probability of detection of a stained spot, carious lesion or crack. These are more prevalent at certain locations, e.g., plaque is typically touching the gum line while stains are often not.

The camera system preferably comprises an illumination source and a detector. As is known, blue light may be used to illuminate the teeth for QLF imaging. This causes the teeth to fluoresce in green (autofluorescence), whereas red fluorescence is caused as a result of the metabolic process of specific bacterial strains.

The image characteristics are for example indicative of a stained spot, carious lesion or crack. These characteristics are more prevalent at certain locations, e.g., plaque is typically touching the gum line while stains are often not.

The system may further comprise an output system configured to provide a notification when plaque is determined to be present with a likelihood above a threshold. This notification is for example used to indicate that a particular area should be brushed more thoroughly to remove the plaque.

The output system may be further configured to provide brushing guidance to a user. This will assist the user in developing a brushing technique which reduces plaque or prevents its formation, by spending more time at plaque vulnerable areas.

The tooth locations may comprise a dentition segment, a specific tooth or a region of a tooth (either a region of a specific tooth or a specific region but of any tooth). The processor is for example configured to apply a segmentation algorithm for detecting a region of a tooth.

For determining a tooth region from images such as QLF images, image segmentation techniques can be used. Artificial intelligence algorithms can be very powerful for this function. Thus, the use of AI is one option for implementation of the segmentation algorithm.

Such algorithms typically require large sets of data with labor-intensive, high-quality annotations. The algorithms are typically trained and run on the cloud, posing privacy and regulatory issues when dealing with personal data. It would be of interest to be able to detect tooth regions, in particular for plaque or tartar detection, without requiring these large computational resources.

As an alternative to the use of AI, a non-intelligent, i.e., non-trained, conventional image processing-based approach (for example comprising intensity-based clustering of pixels and use of a decision tree) may instead be used to provide a semantic segmentation of the tooth regions. This algorithm approach provides the minimally required location information and requires only minimal processing resources. This is particularly relevant when the plaque detection is run real-time off-cloud (for example embedded in an oral care device, or on a local remote device with which the oral care device communicates, such as a mobile phone running an app).

The system may further comprise an inertial measuring unit for monitoring motion of the system thereby to detect the dentition segment (or even individual tooth) at which the system is located. This provides an additional way to detect or confirm the location of the system.

The camera sensor is preferably for generating light induced fluorescence images. In this case, the image characteristics comprise at least red fluorescence.

The invention also provides an oral care device comprising:

> an oral care element; and
> the detection system as defined above.

The detection system may in this way be integrated with an oral care device. Thus, in real time, the device can alert a user to the presence of plaque so that remedial action can be taken. Alternatively, automatic adaptation of the oral care device can be done, based on the detection (without needing an explicit alert to the user). For instance, a brushing intensity setting can be changed, or guidance can be provided to ensure the user brushes the particular location for a longer time. The oral care device is for example a powered toothbrush, wherein the oral care element comprises a driven toothbrush head.

The processor of the powered toothbrush is for example configured to adapt operating parameters of the powered toothbrush in dependence on the determined likelihood of plaque being present.

The invention also provides a method for detecting dental plaque, comprising:

> receiving intra-oral images;
> detecting image characteristics indicative of at least plaque and tartar;
> from the generated images, detect tooth locations at which the image characteristics are present; and
> estimate a likelihood of plaque being present based on the detected image characteristics and their associated tooth locations.

The method for example comprises generating a notification when plaque is determined to be present with a likelihood above a threshold. The method for example comprises detecting a region of a tooth by applying a non-intelligent segmentation algorithm.

The invention also provides a computer program comprising computer program code which is adapted, when said program is run on a processor, to perform the method as defined above.

These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

> Fig. 1 shows a detection system integrated into a power toothbrush;
> Fig. 2 shows a first method of detecting plaque;
> Fig. 3 shows a second method of detecting plaque; and
> Fig. 4 shows an image segmentation and analysis method.

DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a system and method for detecting dental plaque. Intra-oral images are analyzed to detect image characteristics indicative of at least plaque and tartar. Tooth locations are detected at which the image characteristics are present and a likelihood of plaque being present is derived based on the detected image characteristics and their associated tooth locations.

The invention may be a standalone system for detecting plaque, but more preferably it forms part of an oral care device such as an electric power toothbrush.

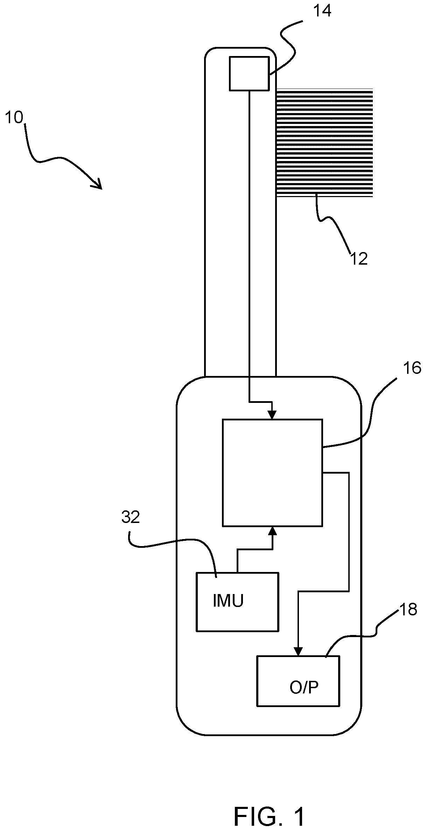

Fig. 1 shows an electric toothbrush 10 having a brush head 12 (which more generally is an oral care element) and a detection system. The detection system is integrated with the toothbrush. The toothbrush is able to alert a user to the presence of plaque so that remedial action can be taken and/or brushing settings of the toothbrush can be changed automatically in response to detection of plaque. The detection system comprises a camera system 14 for generating intra-oral images and a processor 16 for processing the generated images. Image characteristics are identified which are indicative of at least plaque and tartar. In a preferred example, the camera system comprises an illumination source (e.g. a blue light source) and a sensor for recording light-induced fluorescence (QLF) images for QLF detection of plaque. In such a case, the image characteristics comprise red fluorescence.

However, also other imaging-based plaque detection sensors may also benefit from this invention. In such a case, instead of detecting red fluorescence, other optical markers may be detected in the recorded images, associated with plaque and/or tartar.

Furthermore, the image characteristics may be indicative of a stained spot, carious lesion or crack. Thus, while the system of this disclosure is of primary interest for discriminating between plaque and tartar, it also can, in some implementations, estimate a probability of detection of a stained spot, carious lesion or crack.

The generated images are analyzed to detect tooth locations at which the image characteristics are present. By combining the detection of image characteristics and the associated tooth locations, a likelihood of plaque being present can be estimated (and optionally other conditions as mentioned above). The location information enables the system to discriminate between (actionable) plaque and (unactionable) tartar because the probability of each is strongly correlated with location. The location can include dentition segment (e.g., 'inner right upper molars'), specific tooth (e.g., 'upper right 2nd molar'), and /or tooth region (e.g., 'close to gumline').

The dentition segment may signify a particular region within the jaw as well as the relevant tooth surface e.g., the inner (lingual) surface or outer (facial). The dentition (of both jaws) can for example be divided into 16 dentition segments, One segment is the upper front six teeth, and another segment is the lower front six teeth. With two viewing sides, this provides four dentition segments. The four molar/premolar areas each have three viewing sides giving twelve dentition segments. Thus, by this definition, there are 16 dentition segments. Of course, other ways of dividing the overall surfaces of the teeth into sub-regions may be considered.

A specific tooth location may signify a single full tooth surface, and a tooth region location may signify a specific part of the full tooth surface, e.g., at the gum line, interdentally, halfway up the tooth etc. For example, the "Rustogi modification of the Navy plaque index" divides a single tooth surface (facial or lingual) into 9 regions, and this may be used. Of course, other ways of dividing an individual tooth surface into sub-regions may be considered.

ered.

The toothbrush has an output system 18 with the primary purpose of providing a notification when plaque is determined to be present (namely with a likelihood above a threshold). The output system may be a speaker and/or light output. Additionally, or alternatively, it may be a wireless communication system for controlling a remote device such as a smartphone to generate an output message to the user. The output system may also provide brushing guidance to a user. For example, it may adapt existing brushing guidance being provided by a guidance program by increasing the remaining time to brush at the current location when plaque is detected.

The location information is obtained from image analysis, for example using image segmentation techniques. The segmentation algorithm may be a conventional image processing method (as opposed to an AI data-driven method), providing a minimal degree of segmentation required for this application. Additionally, or alternatively, other sensing means can be used. For example, the dentition segment can be detected using an Inertial Measurement Unit (IMU), as currently employed in known electrical toothbrushes which provide user guidance. The IMU data is for example processed by a machine learning algorithm to drive position information.

For the image analysis, a conventional image processing-based approach for example uses intensity-based clustering of pixels and a decision tree. This algorithm approach requires only minimal processing resources which more readily enables the detection to run in real-time off-cloud.

The location information is processed using a database containing probabilities or prevalences of plaque, tartar, and possibly other conditions (as outlined above) for the different tooth locations. Alternatively, a ratio of plaque probability compared to the probability of any other condition leading to the detected image characteristic (e.g. red fluorescence) at the particular locations may be stored. The database may contain additional relevant (meta)data, such as demographic data, health information, etc.

The database may be accessed during use of the algorithm to obtain the location-specific information, or alternatively the database may be used only during training of the algorithm, in the case of a machine learning algorithm, or programming of the algorithm, in the case of a conventional algorithm. Thus, the location-specific information may be embedded in the plaque detection algorithm itself rather than being accessed by the algorithm.

One example will now be explained in which the likelihood of plaque being present is based on using conditional probabilities. Note that the presence of tartar or other relevant features may also be communicated to the user, or brushing guidance or a brush operation mode may also be changed. For example, at a certain level of tartar users may get a recommendation to visit their dental professional.

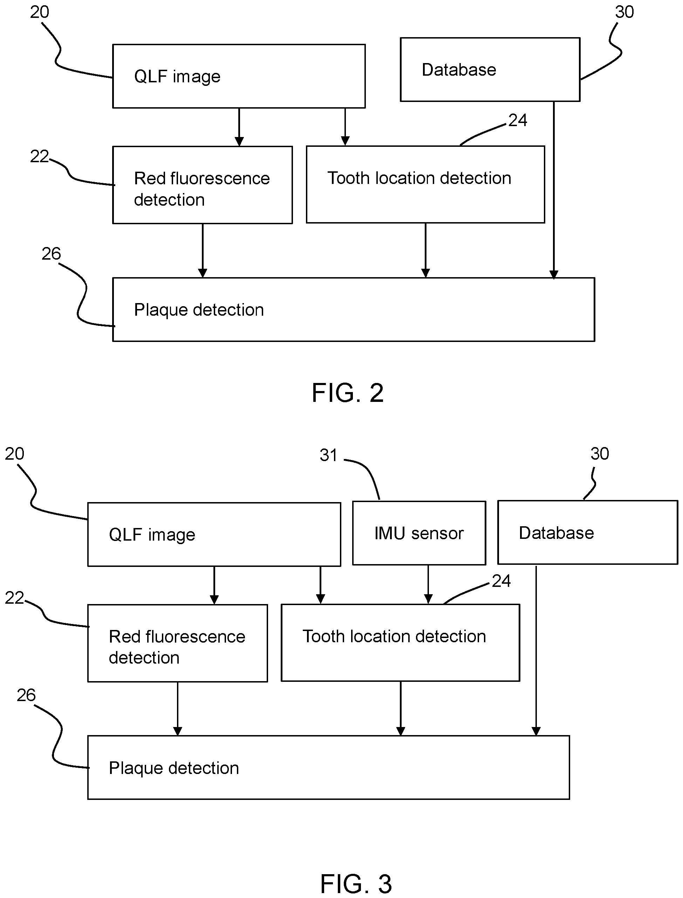

Fig. 2 shows a first example of the detection method applied to a QLF image. The QLF image is received in step 20. From this image, red fluorescence is detected in step 22 and tooth location information is detected in step 24. From this information, together with the databased information 30, plaque detection can take place in step 26.

To explain the method in more detail, it is assumed that a quantitative light-induced fluorescence (QLF) image is obtained from the camera system. Red fluorescence is detected in the image, which can indicate plaque or another condition such as tartar or a stain.

Using statistical data on the prevalence of the various conditions (plaque, tartar, stain, etc.) as a function of tooth location, an output derived from a detection of plaque or a plaque probability is generated.

Conditional probabilities can be employed, such as by Bayes' theorem, which in general form is:

$$P(A|B) = \frac{P(B|A)P(A)}{P(B)},$$

P(A|B) is the probability of A given that B is true, P(B|A) the probability of B given that A is true, P(A) the probability that A is true, and P(B) the probability that B is true. In a concrete example for this application:

$$P(plaque_L|RF_L) = \frac{P(RF_L|plaque_L)P(plaque_L)}{P(RF_L)},$$

$P(plaque_L \mid RF_L)$ is the probability that plaque is present on tooth location L, given that Red Fluorescence (RF) is detected at location L.

$P(RF_L|plaque_L)$ is the probability that Red Fluorescence (RF) occurs at location L if plaque is present at location L. Typically, it may be assumed that this is 100%: i.e., the presence of plaque will always give red fluorescence.

$P(plaque_L)$ is the probability (in general) of plaque occurring on location L. This information is retrieved from the statistical data.

$P(RF_L)$ is the probability that Red Fluorescence occurs (in general) on location L. Here, all conditions leading to Red Fluorescence can be considered (plaque, tartar, stain,...).

If it is assumed that occurrence of any of these conditions gives 100% probability of RF, $P(RF_L)$ becomes the probability that any of the considered conditions occurs on location L. This information is retrieved from the statistical data.

In the above, the ratio of plaque probability on L and the probability of any condition leading to RF occurring on L, $P(plaque_L) / P(RF_L)$, becomes the significant indicator of the presence of plaque.

Hence, in practice the absolute prevalence or probability of plaque and other conditions typically does not need to be known, only the prevalence or probability ratio of plaque and all conditions leading to RF is required.

In a simple example, with statistical data indicating that the probability of plaque occurrence on L is 30%, the probability of tartar on L is 20%, and the probability of any other condition associated with RF is 0%, the ratio $P(plaque_L) / P(RF_L)$ equals 30%(30%+20%) = 0.6 (assuming that plaque and tartar do not occur simultaneously). When red fluorescence is detected, the probability of presence of plaque then equals 60%.

Obviously, highest accuracy may be obtained when the location can be detected with the highest detail: i.e., the location comprises the specific tooth, tooth surface, and tooth region (e.g., 'upper right 2nd molar, inner surface, close to gumline'). However, each of the possible location options is useful stand-alone, for instance:

(i) dentition segment information alone is particularly useful in discriminating plaque from tartar, as red fluorescence on lingual incisor surfaces most likely indicates tartar, whereas on premolars and molars it most likely indicates old plaque;

(ii) tooth region information (independent of the actual tooth) is particularly useful for discriminating between plaque and caries or stains: Red fluorescence occurring isolated on the crown, in the absence of red fluorescence at the gumline or interdentally, most likely indicates caries or stain rather than plaque.

Fig. 3 shows a refinement to the method in which an inertial measurement unit is also used. The same steps are given the same references as in Fig. 2. The IMU sensor data 31 is provided to assist in the tooth location sensing of step 24. The IMU sensor is shown in Fig. 1 as element 32.

It is known to use an IMU for dentition segment detection. For specific tooth detection, a combination of the IMU-based localization (for dentition segment) and image classification may be used. For tooth region, image segmentation is employed. Furthermore, in addition to QLF images, white light images (recorded under 'normal' illumination) may be used. Here, QLF images and white light images may be recorded alternately, using switchable illumination.

Further refinements using the statistical data may also be employed. For instance, metadata, such as demographic data (age, etc.) and health information, can give a more accurate and personalized estimate of the probabilities. Also, personal brushing behavior, e.g., tracked with a connected power toothbrush, can be considered. Furthermore, additional input from dental professionals and inputs from other sensors or tests may be included in the system. For instance, saliva may be tested to see if a person has more likelihood to form tartar.

As explained above, image segmentation may be used for location sensing (i.e., step 24 of Fig. 2 may comprise a segmentation algorithm). This may provide only tooth region detection, without detection of the specific tooth or even dentition segment. Tooth region detection may be based on just the recorded QLF images, using an image segmentation algorithm.

Generally, it is fairly easy to discern teeth from gums and background in QLF images due to their distinct green fluorescence. However, segmentation purely based on color channel intensities may not be sufficient to identify the different tooth regions. Moreover, images can contain (parts of) multiple teeth. Hence, segmentation of the different teeth present in the images is needed before tooth region segmentation.

One example of tooth segmentation algorithm will now be described in more detail, comprising a conventional image processing approach having a set of morphological operations to separate multiple teeth and to associate a tooth region semantic to each pixel in the image.

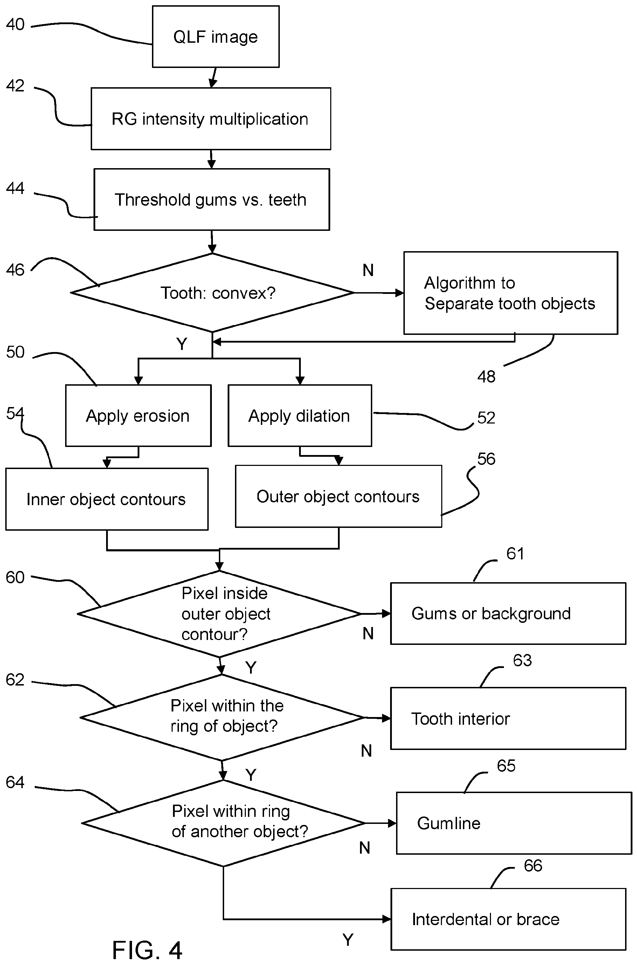

Fig. 4 shows a flowchart for the algorithm.

The QLF image is received in step 40.

For feature selection, the red and green channel pixel intensities are multiplied in step 42. Alternatively, only the green channel intensity may be used.

In step 44 teeth versus non-teeth segmentation is carried out. For instance, Otsu (automatic thresholding) on the red and green channel intensity multiplication results in a black and white map with multiple black and white objects, representing background/gums and teeth respectively. Alternatively, a multi-dimensional clustering algorithm may be used with features such as R*G, R/G, R, G, B pixel intensities.

In steps 46 and 48 there is separation of tooth objects into tooth objects containing a single tooth (or part of a single tooth, e.g., when a brace wire is present which divides the tooth object into two). This involves looping over the tooth objects and checking for convexity in step 46. If the object is (nearly-) convex it likely contains only one tooth.

If not, a watershed algorithm is applied in step 48 to further divide the object into multiple (nearly-) convex objects.

The algorithm then generates inner and outer contours, and a ring for each tooth object. This involves application of erosion in step 50 and dilation in step 52 to each tooth object, to result in polygon inner contours in step 54 and outer contours in step 56.

The dilation and erosion steps enable the interface between the tooth object and its surroundings (another tooth object, or background, or gums) to be determined more robustly. The interface is located somewhere in the ring of the tooth object, where the ring is the region between the inner contour (obtained by erosion) and outer contour (obtained by dilation). The ring is the transitional region around the general outer boundary of the tooth object. This object ring separates the inner region of a tooth object from the background or from the gums or other tooth objects.

Thus, for each tooth object resulting from steps 46 and 48 there is an inner object contour, an outer object contour and a ring. Note that if a brace wire is on a tooth, two tooth objects will be found for that tooth, each with its own inner contour, outer contour, and ring.

Image partitioning is then carried out using the object inner and outer contours, and the location semantic described below is applied to each pixel.

If the pixel is not inside any contour as tested in step 60, the pixel is determined to be in the background or gums in step 61. If the pixel is inside an object outer contour, the method moves to step 62.

If the pixel is not within the ring of the object as tested in step 62, the pixel is determined to be in the inner region of a tooth in step 63 (because it is neither outside the tooth object nor in the transition region i.e., the ring). If the pixel is within the ring of the object, the method moves to step 64. Thus, step 62 concludes that the pixel is in the interior of the tooth if the pixel is not in the ring.

In step 64 it is determined if the pixel is also in the ring of another tooth object. If not, the pixel is determined to be at the gumline or at the interface between the tooth and background (step 65).

If the pixel is also in the ring of another tooth object (so in the ring of two tooth objects and hence where those two tooth objects meet) the pixel is in an interdental region or close to a dental brace wire as determined in step .66. Thus, a pixel in the ring of two tooth objects can equally indicate that the pixel is in an interdental region or close to a brace wire. This is because the algorithm will result in two tooth objects on either side of a brace wire on a single tooth.

The steps above provide a (course) localization of each pixel to determine whether the pixel is on any of four location categories:

> the background or gums;
> the interior of a tooth;
> at or close to the gumline;
> at an interdental area or wire brace area.

In practice, the fact that the segmentation algorithm is unable to distinguish between interdental regions and brace wire regions may be no issue, as both these regions similarly have a relatively higher likelihood of plaque.

Furthermore, the algorithm cannot distinguish between tooth regions close to the gumline and tooth regions at the edge close to the background. However, for better plaque detection accuracy, it can be important to distinguish the gumline. For this purpose, the ring of a tooth object can be further analyzed, for example the edge sharpness and color range may be considered to further discriminate between the tooth-gum interface (i.e., gumline) and the tooth-background interface. Alternatively, or additionally, images recorded under white light and/or near infra-red illumination may be used to discriminate between gum line and non-gumline.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

Functions implemented by a processor may be implemented by a single processor or by multiple separate processing units which may together be considered to constitute a "processor". Such processing units may in some cases be remote from each other and communicate with each other in a wired or wireless manner.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

Any reference signs in the claims should not be construed as limiting the scope.

**Claims**

1. A system for detecting dental plaque, comprising:

   a camera system (14) for generating intra-oral images;
   a processor (16) for processing the generated images, configured to:

      detect image characteristics indicative of at least plaque and tartar;
      from the generated images, detect tooth locations at which the image characteristics are present; and
      estimate a likelihood of plaque being present based on the detected image characteristics and their associated tooth locations.

2. The system of claim 1, wherein the image characteristics are indicative of a stained spot, carious lesion or crack.

3. The system of claim 1 or 2, further comprising an output system (18) configured to provide a notification when plaque is determined to be present with a likelihood above a threshold.

4. The system of claim 3, wherein the output system is further configured to provide brushing guidance to a user.

5. The system of any one of claims 1 to 4, wherein the tooth locations comprise a dentition segment, a specific tooth or a region of a tooth.

6. The system of claim 5, wherein the processor is configured to apply a segmentation algorithm for detecting a region of a tooth.

7. The system of any one of claims 1 to 6, further comprising an inertial monitoring unit (32) for monitoring motion of the system thereby to detect the dentition segment at which the system is located.

8. The system of any one of claims 1 to 7, wherein the camera sensor is for generating light induced fluorescence images.

9. The system of claim 8, wherein the image characteristics comprise red fluorescence.

10. An oral care device comprising:

    an oral care element (12); and
    the system (14,16,18) of any one of claims 1 to 9.

11. The oral care device of claim 10 comprising a powered toothbrush, wherein the oral care element comprises a driven toothbrush head, wherein the powered toothbrush the processor is configured to adapt operating parameters of the powered toothbrush in dependence on the determined likelihood of plaque being present.

12. A method for detecting dental plaque, comprising:

    receiving intra-oral images;
    detecting image characteristics indicative of at least plaque and tartar;
    from the generated images, detect tooth locations at which the image characteristics are present; and
    estimate a likelihood of plaque being present based on the detected image characteristics and their associated tooth locations.

13. The method of claim 12, further comprising generating a notification when plaque is determined to be present with a likelihood above a threshold.

14. The method of claim 12 or 13, comprising detecting a region of a tooth by applying a segmentation algorithm.

15. A computer program comprising computer program code which is adapted, when said program is run on a processor, to perform the method of any one of claims 12 to 14.

14

10

12

16

32

IMU

18

O/P

FIG. 1

FIG. 2

FIG. 3

FIG. 4

| Europäisches Patentamt / European Patent Office / Office européen des brevets | **EUROPEAN SEARCH REPORT** | Application Number EP 23 21 5038 |
|---|---|---|

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2016/125601 A1 (WU YINGQIAN [CN] ET AL) 5 May 2016 (2016-05-05) | 1-3,5,6, 8-15 | INV. A61B5/00 A61C17/22 G06T7/00 |
| Y | * paragraphs [0060], [0066] – [0072], [0091], [0130], [0132], [0160], [0162], [0073], [0116], [0149] – [0151] * | 4,7 | |
| | ----- | | ADD. A46B15/00 |
| X | US 2020/285897 A1 (SHAH PRATIK [US] ET AL) 10 September 2020 (2020-09-10) * paragraphs [0024] – [0026], [0037], [0056] – [0061], [0072], [0075], [0129], [0136] – [0142] * | 1-3,12, 13,15 | |
| | ----- | | |
| Y | EP 3 019 071 A1 (KONINKL PHILIPS NV [NL]) 18 May 2016 (2016-05-18) * paragraphs [0035], [0041] – [0044] * | 4,7 | |
| | ----- | | |
| A | BITTAR DANIELA G ET AL: "Is the red fluorescence of dental plaque related to its cariogenicity?", JOURNAL OF BIOMEDICAL OPTICS, SPIE, 1000 20TH ST. BELLINGHAM WA 98225-6705 USA, vol. 19, no. 6, 1 June 2014 (2014-06-01), page 65004, XP060047413, ISSN: 1083-3668, DOI: 10.1117/1.JBO.19.6.065004 [retrieved on 2014-06-27] * the whole document * | 1-15 | TECHNICAL FIELDS SEARCHED (IPC) A61B A61C A46B A61D G06T |
| | ----- | | |

The present search report has been drawn up for all claims

| Place of search | Date of completion of the search | Examiner |
|---|---|---|
| The Hague | 30 January 2024 | Kowalczyk, Szczepan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 23 21 5038

This annex lists the patent family members relating to the patent documents cited in the above-mentioned European search report.
The members are as contained in the European Patent Office EDP file on
The European Patent Office is in no way liable for these particulars which are merely given for the purpose of information.

30-01-2024

| Patent document cited in search report | Publication date | Patent family member(s) | | Publication date |
|---|---|---|---|---|
| US 2016125601 A1 | 05-05-2016 | NONE | | |
| US 2020285897 A1 | 10-09-2020 | US | 10699163 B1 | 30-06-2020 |
| | | US | 2020285897 A1 | 10-09-2020 |
| EP 3019071 A1 | 18-05-2016 | CN | 105407793 A | 16-03-2016 |
| | | EP | 3019071 A1 | 18-05-2016 |
| | | JP | 6371842 B2 | 08-08-2018 |
| | | JP | 2016523668 A | 12-08-2016 |
| | | RU | 2016104399 A | 16-08-2017 |
| | | US | 2017000352 A1 | 05-01-2017 |
| | | WO | 2015003939 A1 | 15-01-2015 |

EPO FORM P0459

For more details about this annex : see Official Journal of the European Patent Office, No. 12/82